# EUROPEAN PATENT APPLICATION

(11) **EP 3 120 867 A1**
(43) Date of publication of application: **25.01.2017**
(21) Application number: 15178301.6
(22) Date of filing: 24.07.2015
(51) Int. Cl.: A61K 38/48, A61P 25/00

(54) **NEUROTOXIN FOR USE IN THE TREATMENT OF A PERSONALITY DISORDERS**

(71) Applicant: Medizinische Hochschule Hannover, 30625 Hannover (DE); Asklepios Kliniken Hamburg GmbH, 22307 Hamburg (DE); Magid, Michelle, Austin, TX 78731 (US)
(72) Inventor: Krüger, Prof. Dr. med., Tillmann, 30161 Hannover (DE); Wollmer, PD Dr. med., M. Alex, 22041 Hamburg (DE); Magid, Michelle, Austin, Texas 78731 (US)
(74) Representative: Taruttis, Stefan Georg

(57) **Abstract**

The present invention relates to the use of a neurotoxin, especially botulinum toxin, for use as a medicament in the treatment of a personality disorder and/or a habit and impulse disorder and/or a conduct disorder, as well as to a method for the treatment of a personality disorder and/or of a habit and impulse disorder and/or of a behavioural and emotional disorder with onset usually occurring in childhood and adolescence by administration of the neurotoxin to a patient diagnosed with this disorder. Preferably, the neurotoxin is for use as a medicament in the treatment of an emotionally unstable personality disorder and/or of another personality disorder and/or of a habit and impulse control and/or behavioural and emotional disorders with onset usually occurring in childhood and adolescence, in which the disorder is preferably characterised by emotional instability and impulsivity.

## Description

The present invention relates to the use of a neurotoxin, especially botulinum toxin for use as a medicament in the treatment of a personality disorder and/or a habit and impulse disorder and/or a conduct disorder, as well as to a method for the treatment of a personality disorder and/or of a habit and impulse disorder and/or of a behavioural and emotional disorder with onset usually occurring in childhood and adolescence by administration of the neurotoxin to a patient diagnosed with this disorder. Preferably, the neurotoxin is for use as a medicament in the treatment of an emotionally unstable personality disorder and/or of another personality disorder and/or of a habit and impulse control and/or behavioural and emotional disorders with onset usually occurring in childhood and adolescence, in which the disorder is preferably characterised by emotional instability and impulsivity.

The neurotoxin preferably is botulinum toxin, also termed botulinum neurotoxin (BoNT), preferably BoNT-A or BoNT-B, but also any other type of BoNT, components, analogues, derivatives, or fragments thereof, capable to induce a circumscribed and sustained relaxation of a facial muscle. The neurotoxin, e.g. BoNT and the other compounds, are preferably for local administration, preferably for local administration by injection into the targeted muscle or injection into the connective tissue in the vicinity of the targeted muscle or to the skin surface above the targeted muscle in a dose for sustained relaxation or paralysis of the targeted muscle. The dose necessary for sustained relaxation or paralysis of a targeted muscle can be determined by the skilled person.

### State of the art

WO 2005/084705 A1 describes the use of the neurotoxin botulinum toxin as a medicament in the treatment of neurological disorders by injecting botulinum toxin into a trigeminal sensory nerve or to the vicinity of a trigeminal sensory nerve, especially by non-intramuscular injection for paralyzing the sensory trigeminal nerve or its branches. In addition to the treatment of neurological disorders botulinum toxin is used for the treatment of neuropsychiatric disorders, including schizophrenia, Alzheimer's disease, mania and anxiety, mood disorders, in particular bipolar disease, and movement disorders.

US 2015/0132282 A1 describes a method for treating social anxiety disorder, obsessive compulsive disorder or panic disorder by administration of a neural toxin to the corrugator supercillii and/or procerus muscle to cause their paralysis.

Wollmer et al., Journal of Psychiatric Research pp 574-581 (2012), describe the treatment of depression by injection of botulinum toxin into the glabella frown lines. The study selected patients that were able to produce an at least moderate glabella line at maximum frowning.

### Object of the invention

It is an object of the invention to provide a medicament and a method for treatment using the medicament for the treatment of personality disorders and/or habit and impulse control disorders and/or behavioural and emotional disorders with onset usually occurring in childhood and adolescence which are disorders that are characterized by emotional instability and impulsivity, especially for the emotionally unstable personality disorder,

### Description of the invention

The invention achieves the objects by the features of the claims, especially by providing a neurotoxin, especially botulinum toxin for use as a medicament in the treatment of a personality disorder and/or a habit and impulse control disorder and/or behavioural and emotional disorders with onset usually occurring in childhood and adolescence, wherein the treatment is application of a neurotoxin, especially botulinum toxin, for paralysing at least one of the muscles of the face, of the head and/or of the neck which are related to negative emotions, preferably emotional instability and/or impulsivity, preferably into at least one of the muscles of the face. Further, the invention relates to a process for the treatment of the personality disorder and/or a habit and impulse control disorder and/or behavioural and emotional disorders with onset usually occurring in childhood and adolescence by intra-muscular injection of the neurotoxin. Paralysing of the at least one muscle can e.g. be by use of the neurotoxin for intra-muscular injection or for topical application to at least one muscle.

According to the invention, paralysis of the muscles of the face, of the head and/or of the neck which are related to negative emotions and behaviours can be achieved by intra-muscular injection or by topical application of the neurotoxin. These applications of the neurotoxin to the muscles of the face, of the head and/or of the neck which are related to negative emotions preferably only paralyse at least one muscle.

The negative emotions and behaviours can e.g. be selected from the basic emotions including sadness, anger, fear and disgust and from combinations of at least two of these. Additionally, negative emotions can be found among complex emotions including anguish, contempt, distrust, envy, guilt, hate, jealousy, shame and from combinations of at least two of these. Finally, different other emotions and psychopathological features can occur such as aggressiveness, agitation, ambivalence, ambitendence, akathisia, affective lability, affective incontinence, affective rigidity, anxiety, episodic dysphoria, histrionics, irratibility, affective/emotional instability, inner restlessness, logorrhea, mannerisms, motor restlessness, impulsivity/impulsiveness, parakinisia, self-mutilation, stuttering, trichotillomania, nail-biting, uncooperativeness.

Preferred muscles (M.) are M. alaequae nasi, M. buccinator, M. corrugator supercillii, M. depressor supercillii, M. depressor anguli oris, M. frontalis, M. levator anguli oris, M. levator labii superioris M. masseter, M. mentalis, M. nasalis, M. transversus menti, M. orbicularis oris, M. orbicularis occuli, Platysma, M. procerus, M. temporalis as well as all muscles responsible for movement of head, neck and shoulders. More preferred the corrugator and procerus muscle, as the activation of these muscles discriminates most precisely between negative (active) and not-negative (inactive) emotions. But also other facial muscles that are involved in the expression of negative emotions can be the target for injection of the neurotoxin.

Muscles related to negative emotions and behaviours in general are muscles which in negative emotions/behaviours are contracted, e.g. in negative emotions such as those occurring in at least one of a specific personality disorder, especially in an emotionally unstable personality disorder, in a mixed and other personality disorder, in an enduring personality change, not attributable to brain damage and disease, or in a habit and impulse disorder or behavioural and emotional disorders with onset usually occurring in childhood and adolescence, including their subgroups, and combinations thereof.

Herein, the disorders are characterized and classified in accordance with ICD-10 (World Health Organisation (WHO), 2015). The disorders for treatment by the use of the neurotoxin of the invention are classified as disorders of adult personality and behaviour in F60, F61, F62 or F63. These disorders are generally characterized by a variety of conditions and behaviour patterns of clinical significance which tend to be persistent and appear to be the expression of the individual's characteristic lifestyle and mode of relating to himself or herself and others. Some of these conditions and patterns of behaviour emerge early in the course of individual development, as a result of both constitutional factors and social experience, while others are acquired later in life. Specific personality disorders (F60), mixed and other personality disorders (F61), and enduring personality changes (F62) are deeply ingrained and enduring behaviour patterns, manifesting as inflexible responses to a broad range of personal and social situations. They represent extreme or significant deviations from the way in which the average individual in a given culture perceives, thinks, feels and, particularly, relates to others. Such behaviour patterns tend to be stable and to encompass multiple domains of behaviour and psychological functioning. They are frequently, but not always, associated with various degrees of subjective distress and problems of social performance.

The preferred indication for use of the neurotoxin is the emotionally unstable personality disorder classified as F60.3 in ICD10, which in DSM 5 is referred to as borderline personality disorder.

Generally, the disorders for treatment by the use of neurotoxin according to the invention are characterized by emotional instability in combination with impulsivity, especially in adult persons.

The emotionally unstable personality disorder is characterized by the following characteristics:
A pervasive pattern of instability of interpersonal relationships, self-image, affects as well as marked impulsivity, beginning by early adulthood and present in a variety of contexts as indicated by at least five of the following characteristics (DSM-5):
   1. Frantic efforts to avoid real or imagined abandonment.
   2. A pattern of unstable and intense interpersonal relationships characterised by alternating between extremes of idealisation and devalution.
   3. Identity disturbance: markedly and persistently unstable self-image or sense of self.
   4. Impulsivity in at least two areas that are potentially self-damaging.
   5. Recurrent suicidal behaviour, gestures, or threats, or self-mutilating behaviours.
   6. Affective instability due to a marked reactivity of mood (e.g. intense episodic dysphoria, irritability, or anxiety usually lasting a few hours and only rarely more than a few days.
   7. Chronic feelings of emptiness.
   8. Inappropriate, intense anger or difficulty controlling anger (e.g. frequent displays of temper, constant anger, recurrent physical fights).
   9. Transient, stress-related paranoid ideation or severe dissociative symptoms.

The essential feature of the emotionally unstable/borderline personality disorder is a pervasive pattern of instability of interpersonal relationships, self-image, and affects, and marked impulsivity that begins by early adulthood and is present in a variety of contexts. The individual person may be very sensitive to environmental circumstances. The clinical key features are (1) affective/emotional instability and (2) impulsivity, which can also be found in other personality disorders for treatment by the use of neurotoxin according to the invention, such as a personality disorder of cluster A, B and C, e.g. paranoid (F60.0), antisocial (F60.2), histrionic (F60.4), obsessive-compulsive (F60.5), avoidant (F60.6), narcissistic disorder (F60.80), passive-aggressive (F60.81), as well as disruptive, impulse-control (e.g. pyromania (F63.1), kleptomania (F63.3), intermittent explosive disorder (F63.81)) and conduct disorders, i.e. behavioural and emotional disorders with onset usually occurring in childhood and adolescence (F 90-98), especially conduct disorders (F91.1, F91.2), oppositional defiant disorder F91.3, other specified and unspecified disruptive, impulse-control, and conduct disorder (F91.8 & F91.9); tic disorders (F95) in particular tic (F95.1) and Tourette syndrome (F95.2) and other behavioural and emotional disorders with onset usually occurring in childhood and adolescence (F98), in particular stuttering (F98.5) and (F98.6).

Affective/emotional instability in emotionally unstable personality disorder is often due to marked reactivity of mood (e.g. intense episodes of dysphoria, irritability, or anxiety usually lasting a few hours and only rarely more than a few days). Basic dysphoric mood can be disrupted by periods of other intense emotions such as anger, panic, or despair and is rarely relieved by periods of well-being or satisfaction. These episodes may reflect the individual's extreme reactivity to interpersonal stresses. Affected individuals frequently express inappropriate, intense anger and tension or have difficulty controlling their anger and tension. In general in the disorders for treatment according to the invention, and especially in the emotionally unstable personality disorder, impulsivity is found in at least two areas that are potentially self-damaging such as gambling, spending money irresponsibly, binge eating, abusing substances, engaging in unsafe sex, or driving recklessly. In addition, impulsive behaviours may comprise aggressive (towards oneself or towards others) or risky actions prompted by negative emotions.

Generally preferred, the characteristics of lack of drive, predominant, severe and ongoing anxiety or panic, depressed mood, delusions, hallucinations, disturbances of cognitive function indicating e.g. mood disorders (major depressive disorders, anxiety disorders), schizophrenia or dementias are not key features or not present.

The characteristics are determined in patients who can be classified as specific personality disorders (F60), including F60.0 to F60.9, who can be classified as mixed and other personality disorders (F61), who can be classified as enduring personality changes not attributable to brain damage and disease (F62), or who can be classified as habit and impulse disorders (F63), characterized behavioural and emotional disorders with onset usually occurring in childhood and adolescence (F90-98).

Preferably, the emotionally instable personality disorder (F60.3; impulsive type F60.30; borderline type F60.31) is characterized by the following additional characteristics: Excess and lack of control of negative emotions and by impulsive behaviour arising thereof. Also included are other personality and habit and impulse control and behavioural and emotional disorders with onset usually occurring in childhood and adolescence (F90-98) that are characterised by an access of negative emotions, emotional instability or impulsive behaviour like paranoid (F60.3), dissocial (F60.2), histrionic (F60.4), anxious-avoiding (F60.6), or narcissistic (F60.80) personality disorder, as well as combined personality disorder consisting of at least two of the former personality disorder (F61.0), persistent personality changes characterised by an increase in the prevalence of negative emotions or impulse behavior (F62) and disorders of impulse control in the basis of abundant negative emotions (F63).

The emotionally unstable personality disorder is characterized by a definite tendency to act impulsively and without consideration of the consequences; the mood is unpredictable and capricious (ICD-10). There is a liability to outbursts of emotion and an incapacity to control the behavioural explosions. There is a tendency to quarrelsome behaviour and to conflicts with others, especially when impulsive acts are thwarted or censored. Two types may be distinguished in emotionally unstable personality disorder: the impulsive type, characterized predominantly by emotional instability and lack of impulse control, and the borderline type, characterized in addition by disturbances in self-image, aims, and internal preferences, by chronic feelings of emptiness, by intense and unstable interpersonal relationships, and by a tendency to self-destructive behaviour, including suicide gestures and attempts.

This combination of characteristics is determined in patients who can be classified as emotionally unstable personality disorders of F60.3, including the aggressive personality disorder, the borderline personality disorder and the explosive personality disorder.

In contrast to the personality disorder and/or habit and impulse control disorder and/or behavioural and emotional disorders with onset usually occurring in childhood and adolescence which are treated by use of the neurotoxin according to the invention, affective disorders, schizophrenic and psychotic disorders and dementia are excluded.

Preferably, the neurotoxin is for use in psychiatry and psychotherapy for the intra-muscular injection/topical application in the treatment of the personality disorder. Therein, psychiatric and psychotherapeutic treatment comprises or consists of the following steps: Clinical assessment, psychoeducation, skills training, interpersonal training, social competence training, self-help, and pharmacological treatment of comorbid psychiatric disorders. For emotionally unstable personality disorders there is currently no approved medication available.

One surprising advantage of the use of the neurotoxin according to the invention is that the therapeutic effect against the personality disorder and/or habit and impulse disorder and/or behavioural and emotional disorders with onset usually occurring in childhood and adolescence is independent from the occurrence of visible muscle contraction of the muscles associated with negative emotions and is primarily related to negative emotions, affective/emotional irritability and impulsivity. Therefore, the neurotoxin for use in the treatment allows to treat patients who are diagnosed with such a personality disorder and/or habit and conduct disorder and/or behavioural and emotional disorders with onset usually occurring in childhood and adolescence independent from the presence of visible or significant disorder-related contractions of muscles associated with negative emotions, e.g. patients who are free from persistent or disorder-related muscle contractions of muscles associated with negative emotions, e.g. of patients who are free from visible glabella frown lines.

Another advantage of the use of the neurotoxin according to the invention is that the administration of the neurotoxin results in a therapeutic effect that at least to a significant extent persists for the duration of the paralysis of the muscle associated with negative emotions, e.g. for at least three, preferably up to six months. This persistence of the therapeutic effect allows for long intervals of therapy without further administration of the neurotoxin.

On the example of the emotionally unstable personality disorder, it has been found that disorders of adult personality and behaviour disorders classified as F60 to F63 of ICD-10 (WHO) personality disorders can be treated by intramuscular injection into the muscles of the head, neck and face which are related to negative emotions. This treatment has been found to have a significant effect in patients even in the absence of visible contractions of these muscles, e.g. in a treatment by injection of the neurotoxin into the corrugator supercillii muscle and/or procerus muscle in the absence of visible glabella lines. In these applications of the neurotoxin, the sensory trigeminal nerve was not paralysed. The invention is now described in greater detail by way of examples.

### Example 1: Treatment of emotionally unstable personality disorder

One female patient, 59 years of age, diagnosed with emotionally unstable personality disorder and comorbid alcohol dependence and major depressive episode, having symptoms of emotional instability, impulsivity and mood swings came for admission for treatment of emotionally unstable personality disorder. The patient had received different in- and outpatient treatments including psychotherapy and pharmacotherapy without a significant improvement of the condition. After written informed consent the patient was administered five injections into the corrugator supercillii and procerus muscle at 30 units Onabotulinumtoxin A (available under the trademarks Vistabel and Botox), distributed to five injection sites. Within four weeks borderline symptoms improved on the Borderline Symptom List (BSL-23) with an initial score of 68 and 42 after treatment as diagnosed by professional clinical psychiatric assessment.

### Example 2: Treatment of emotionally unstable personality disorder and social phobia

A 27 year old female patient diagnosed with emotionally unstable personality disorder and comorbid social phobia received specialized inpatient treatment in sense of a dialectic behavioural therapy (DBT). However, at the end of treatment there was no response. After written informed consent the patient was then administered with five injections into the corrugator supercillii and procerus muscle at 30 units Onabotulinumtoxin-A (available under the trademarks Vistabel and Botox), distributed to five injection sites. Within four weeks borderline symptoms on the BSL-23 significantly decreased from 76 to 26 points, week 6: 22 points. After three months there was a recurrence of symptoms and the BSL-23 score again increased with values of 62 (3 months), 78 (4 months) and 79 (5 months). She then received another BTX injection as described above and the BSL-23 score again decreased: 69 after four weeks, 43 after two months, and 25 after three months as diagnosed by professional clinical psychiatric assessment.

### Example 3: Treatment of emotionally unstable personality disorder with comorbid depression

A 26 year old female patient with comorbid depression received inpatient treatment. As there was no response she then received treatment with BTX as outlined above in Example 2. After BTX injection there was an improvement of BSL-23 score from initially 73 to 14 after six weeks. Depressive symptoms also improved, however, not as convincingly as borderline symptoms as diagnosed by professional clinical psychiatric assessment.

### Example 4: Treatment of emotionally unstable personality disorder

A 20 year old patient with emotionally unstable personality disorder received inpatient treatment (DBT) without sufficient success. She was then offered BTX treatment as outlined above. After BTX injection borderline symptoms significantly improved with BSL-23 scores of initially 72 to 13 after four weeks and 7 after two months. Similarly, expert rating improved as assessed by the Zanarini-BPD-Scale from initially 17 to 1 after four weeks and 0 after two months as diagnosed by professional clinical psychiatric assessment.

### Example 5: Treatment of enduring personality change after catastrophic experience (F62.0) and posttraumatic stress disorder (PTSD)

A 41 year old female patient with an enduring personality change after catastrophic experience (F62.0) in terms of emotionally unstable personality symptoms and posttraumatic stress disorder as well as comorbid depressive episodes received inpatient treatment without sufficient success. Previously, she was placed in a psychiatric home after she was not able to fulfil her social roles as mother (2 children) and wife. Most importantly, she regularly depicted states of dissociation during which she committed severe self-mutilation and behavioral disturbances including injuries of nerves and vessels, drops on the floor and knocking her head against walls and floors. She then desired treatment as outlined above in Example 2. After two weeks already, expert rating (Zanarini-BPD-Scale) documented marked improvement with a reduction of the score from 23 to 8, and this remained stable after four and eight weeks. Notably, a significant improvement of dissociative states was observed with less or even no self-harming. Comorbid symptoms also improved. In concordance with her family she then even decided to move back from the psychiatric home to her family and their private home. She received two additional BTX injections about every four months when some of the symptoms reoccurred as diagnosed by professional clinical psychiatric assessment.

### Example 6: Treatment of therapy resistant major depression disorder and comorbid stutter (F98.5)

A 40 year old male patient presented himself in the clinical department of the applicant with a chronic (more than two years) therapy resistant (more than two different treatments without response) major depressive disorder. Most importantly, he also suffered from stutter since childhood which never improved (F98.5). Before a number of different drugs as well as additional biological treatment approaches have been tried including irreversible monoamine oxidase inhibitors (MAOI), electro convulsive therapy (ECT), vagal nerve stimulation, transcranial magnetic stimulation and others. After written informed consent the patient was administered with five injections into the corrugator supercillii and procerus muscle at 30 units Onabotulinumtoxin-A (available under the trademarks Vistabel and Botox), distributed to five injection sites. After four weeks a significant improvement of mood as assessed by the Beck Depression Inventory (BDI, self-rating) and the Hamilton Depression Rating Scale (HAMD, expert rating) was observed. This was a relief after many years of chronic depression. Moreover, symptoms of stutter significantly improved as noticed by the patient and professionals and flow of words and conversation was much better than before as diagnosed by professional clinical psychiatric assessment.

## Claims

1. Neurotoxin for use as a medicament for paralysis of at least one of the muscles of the face, of the head and of the neck which is associated with negative emotions in the treatment of a personality disorder that is **characterised by** impulsivity in combination with emotional instability.

2. Neurotoxin for use as a medicament according to claim 1, **characterized in that** the neurotoxin is for intra-muscular injection of botulinum toxin into the muscles.

3. Neurotoxin for use as a medicament according to claim 1, **characterized in that** the neurotoxin is for topical application.

4. Neurotoxin for use as a medicament according to one of the preceding claims, **characterized in that** the neurotoxin is botulinum toxin.

5. Neurotoxin for use as a medicament according to one of the preceding claims, **characterised in that** the muscle is selected from the group comprising or consisting of M. buccinator, M. corrugator supercillii, M. depressor supercillii, M. depressor anguli oris, M. frontalis, M. levator anguli oris, M. masseter, M. mentalis, M. nasalis, M. transversus menti, M. orbicularis oris, M. orbicularis occuli,, Platysma, M. procerus, M. temporalis as well as all muscles responsible for movement of head, neck and shoulders, M. frontalis, M. nasalis, M. levator labii superioris, M. alaequae nasi, M. depressor anguli oris, and combinations of at least two of these.

6. Neurotoxin for use as a medicament according to one of the preceding claims, **characterized in that** paralysis is effective to only paralyse one or more muscles of the muscles selected from the group comprising or consisting of M. procerus, M. corrugator supercillii, M. depressor anguli oris, M. mentalis, M. nasalis, M. levator anguli oris, M. frontalis, M. levator labii superioris, M. alaequae nasi, and combinations of at least two of these.

7. Neurotoxin for use as a medicament according to one of the preceding claims, **characterized in that** the personality disorder is **characterized by** a definite tendency to act impulsively and without consideration of the consequences; the mood is unpredictable and capricious, and with a liability to outbursts of emotion and an incapacity to control the behavioural explosions.

8. Neurotoxin for use as a medicament according to one of the preceding claims, **characterized in that** the personality disorder is a paranoid, antisocial, histrionic, narcissistic, avoidant, obsessive-compulsive disorder, a disruptive disorder, impulse-control disorder or conduct disorder or behavioural and emotional disorders with onset usually occurring in childhood and adolescence.

9. Neurotoxin for use as a medicament according to one of the preceding claims, **characterized in that** the personality disorder is **characterized by** a variety of conditions and behaviour patterns of clinical significance which tend to be persistent and appear to be the expression of the individual's characteristic lifestyle and mode of relating to himself or herself and others, wherein some of these conditions and patterns of behaviour emerge early in the course of individual development, as a result of both constitutional factors and social experience, while others are acquired later in life, which personality disorder is a specific personality disorder (F60), mixed and other personality disorders (F61), or an enduring personality change (F62), which is a deeply ingrained and enduring behaviour pattern, manifesting as inflexible responses to a broad range of personal and social situations and representing extreme or significant deviations from the way in which the average individual in a given culture perceives, thinks, feels and, particularly, relates to others, which behaviour pattern tends to be stable and to encompass multiple domains of behaviour and psychological functioning, and frequently, but not always, associated with various degrees of subjective distress and problems of social performance.

10. Neurotoxin for use as a medicament according to one of the preceding claims, **characterized in that** the personality disorder is **characterized by** a pervasive pattern of instability of interpersonal relationships, self-image, affects as well as marked impulsivity, beginning by early adulthood and present in a variety of contexts as indicated by at least five of the following characteristics (DSM-5):
1. Frantic efforts to avoid real or imagined abandonment
2. A pattern of unstable and intense interpersonal relationships **characterised by** alternating between extremes of idealisation and devaluation
3. Identity disturbance: markedly and persistently unstable self-image or sense of self.
4. Impulsivity in at least two areas that are potentially self-damaging
5. Recurrent suicidal behaviour, gestures, or threats, or self-mutilating behaviours.
6. Affective instability due to a marked reactivity of mood (e.g. intense episodic dysphoria, irritability, or anxiety usually lasting a few hours and only rarely more than a few days.
7. Chronic feelings of emptiness.
8. Inappropriate, intense anger or difficulty controlling anger (e.g. frequent displays of temper, constant anger, recurrent physical fights).
9. Transient, stress-related paranoid ideation or severe dissociative symptoms.

11. Neurotoxin for use as a medicament according to one of the preceding claims, **characterized in that** the personality disorder is an emotionally unstable personality disorder (F60.3), especially the borderline personality disorder classified in F60.3 of ICD10.

12. Neurotoxin for use as a medicament according to one of the preceding claims, **characterized in that** the negative emotion is selected from the basic emotions including sadness, anger, fear and disgust and from combinations of at least two of these. Additionally, negative emotions can be found among complex emotions including anguish, contempt, distrust, envy, guilt, hate, jealousy, shame and from combinations of at least two of these. Finally, different other emotions and psychopathological features can occur such as aggressiveness, agitation, ambivalence, ambitendence, akathisia, affective lability, affective incontinence, affective rigidity, anxiety, episodic dysphoria, histrionics, irratibility, affective and/or emotional instability, inner restlessness, logorrhea, mannerisms, motor restlessness, impulsivity/impulsiveness, parakinisia, self-mutilation, stuttering, trichotillomania, nail-biting, uncooperativeness.

13. Neurotoxin for use as a medicament according to one of the preceding claims, **characterized in that** it is for use in a psychiatric and/or psychotherapeutic treatment.

14. Neurotoxin for use as a medicament according to one of the preceding claims, **characterized in** excluding injections into a ganglion of the sensory trigeminal nerve and excluding injections into the vicinity of a ganglion of the sensory trigeminal nerve resulting in exclusion of paralysis of a ganglion of the sensory trigeminal nerve.

15. Neurotoxin for use as a medicament according to one of the preceding claims, **characterized in that** the personality disorder typically is not **characterized by** severe lack of drive, predominant, severe and ongoing anxiety or panic, severely depressed mood, delusions, hallucinations, disturbances of cognitive function, schizophrenia or dementia.
